# EUROPÄISCHE PATENTANMELDUNG

(11) **EP 0 529 510 A1**
(43) Veröffentlichungstag der Anmeldung: **03.03.1993**
(21) Anmeldenummer: 92114187.5
(22) Anmeldetag: 20.08.1992
(51) Int. Cl.: A61N 1/30

(54) **Kontrollierte transdermale iontophoretische Applikation pharmakologisch aktiver Wirkstoffe**

(30) Priorität: 23.08.1991 DE 4127951
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Rohr, Uwe, Dr., W-6507 Ingelheim (DE); Leonhardt, Friedrich, W-6507 Ingelheim/Rhein (DE); Wolf, Manfred, Dr., W-6531 Schöneberg (DE)

(57) **Zusammenfassung**

Die Erfindung betrifft ein Verfahren zur kontrollierten iontophoretischen Applikation von Wirkstoffen und dafür geeignete Vorrichtungen.

## Beschreibung

Die Erfindung betrifft ein Verfahren zur kontrollierten iontophoretischen Applikation von Wirkstoffen und dafür geeignete Vorrichtungen.

Die Iontophorese ist eine seit langem bekannte Methode, um ionische Wirkstoffe unter Anlegung eines elektrischen Feldes durch die intakte Haut oder andere Körperflächen zu applizieren. In einer einfachen Ausführungsform bestehen zur Iontophorese geeignete Vorrichtungen aus zwei Elektroden, von denen mindestens eine den Wirkstoff enthält und einer Energieversorgung zum Aufbau des elektrischen Feldes. Weiter entwickelte Ausführungsformen ermöglichen es, die Zeit und Dauer des angelegten elektrischen Feldes zu programmieren, mit dem Ziel, die Dosierung der Arzneistoffmenge den individuellen Bedürfnissen des Patienten anzupassen.

Überraschenderweise hat sich herausgestellt, daß die bislang bekannten iontophoretischen Vorrichtungen nicht dazu geeignet sind, das vorgegebene Ziel, die exakte vorgegebene Wirkstoffmenge individuell einzuhalten.

Zu Beginn der Therapie, d.h., sobald der Stromkreis zu den Elektroden geschlossen ist, beginnt der Wirkstoff durch die Haut zu migrieren und gelangt nach kurzer Zeit in das Blutgefäßsystem. Je nach Stärke des elektrischen Feldes und den physikochemischen Eigenschaften des Wirkstoffes werden sehr schnell Blutspiegelwerte erreicht, die denen einer intravenösen Applikation entsprechen. Entgegen den Erwartungen wird jedoch die Zufuhr an Wirkstoff durch die. Haut nicht sofort unterbrochen, sobald die Stromzufuhr zu den Elektroden unterbrochen wird. In einigen Fällen wurde noch 30 Stunden nach Abschalten der Stromzufuhr eine unverminderte Wirkstoffzufuhr - resultierend in gleichbleibenden Plasmaspiegeln - beobachtet. Dies Zeigt, daß die bislang bekannten iontophoretischen Systeme nur bedingt zum Einsatz als "on demand" Systeme geeignet sind, da eine genaue Zeit/Dosis kontrollierte Wirkstoffapplikation nicht möglich ist.

Erfindungsgemäß wird ein Verfahren zur iontophoretischen Applikation von Wirkstoffen vorgeschlagen, daß dadurch gekennzeichnet ist, daß ein geeigneter Wirkstoff für eine vorherbestimmte Zeit unter Anwendung eines elektrischen Feldes perkutan appliziert wird und anschließend ein entgegengesetzt geladenes elektrisches Feld angelegt wird, wodurch der Arzneimittelfluß aus der wirkstoffhaltigen Elektrode gestoppt wird. Bedingt durch das elektrische Gegenfeld wird auch der Anteil an Wirkstoff, der infolge der Migration sich noch in den obersten Hautschichten befindet, zurückgehalten, so daß der Plasmaspiegel nicht weiter ansteigt und kontrollierbar bleibt.

Gegenüber dem Stand der Technik ist es mit dem erfindungsgemäßen Verfahren möglich, den Arzneimittelflux zeitlich exakt zu kontrollieren und einzustellen und somit die aufzunehmende Wirkstoffmenge genau einzuhalten.

Weiterhin ist es möglich, durch eine definierte Pulsfolge der Wirkstoffapplikation - jeweils unterbrochen von Zeiteinheiten, in denen das Gegenfeld angelegt ist - Blutplasmaspiegel aufzubauen, ohne daß ein vorgegebener maximaler Wert des Blutplasmaspiegels infolge eines Aufschwingen des Systems überschritten wird.

Von besonderem Vorteil ist das erfindungsgemäße System bzw. Verfahren bei der iontophoretischen Applikation von Wirkstoffen, die ein hohes passives Diffusionsvermögen aufweisen und üblicherweise in einfachen transdermalen Systemen eingesetzt werden. Hierzu zählen Moleküle, die im Wirkstoffreservoir nicht in ionischer Form vorliegen und erst durch Anlegen des elektrischen Feldes polarisiert werden. Solche Moleküle weisen ein wesentlich stärkeres passives Diffusionsvermögen (Penetration) auf als ionische, so daß nach Abschalten des elektrischen Feldes die Nachteile der ungewollten und eventuell unkontrollierten Diffusion in das Blutgefäßsystem wesentlich größer sind, als bei in ionischer Form vorliegenden Molekülen.

In dieser Beschreibung wird der Begriff "Migration" im Sinne des durch das elektrische Feld bedingten Wirkstofftransports gebraucht, der Begriff "Penetration" entspricht der passiven Diffusion des Wirkstoffs durch die Haut ohne eines angelegten elektrischen Feldes, wie es der üblichen passiven transdermalen Applikation entspricht.

Die Vorrichtung zur Durchführung des erfindungsgemäßen Verfahrens besteht im wesentlichen auf folgenden Merkmalen:
a) eine wirkstoffhaltige Elektrode,
b) eine wirkstofffreie Gegenelektrode,
c) eine Vorrichtung zur Stromversorgung, die mit den beiden Elektroden verbunden ist, so daß bei angelegter Spannung zwischen den beiden Elektroden ein elektrisches Feld aufgebaut wird, wobei die Elektroden in Kontakt mit der Haut des Patienten sind,
d) eine Vorrichtung, die es ermöglicht, die Polarität der Elektroden und damit die des elektrischen Feldes umzukehren, so daß unmittelbar nach der Umkehr der Polarität die Migration und Diffusion des Wirkstoffes gestoppt wird.

Auch wenn es in einer einfachsten Ausführungsform möglich ist, daß das Ein- und Ausschalten der Stromversorgung wie auch die Umkehrung der Polarität rein mechanisch erfolgen kann, so ist es doch bevorzugt, entsprechende mikroelektronische Bauteile mit integrierten Schaltungen mit variabler Steuerung zu verwenden.

Eine elektronische Zeitkontrolle ermöglicht das Einschalten des Feldes zu einem beliebig vorwählbaren Zeitpunkt. Nach einer vorgegebenen Zeitspanne, in der der Patient die gewünschte Menge an Wirkstoff aufgenommen hat (diese kann individuell unterschiedlich sein), und die entsprechend vorprogrammiert wurde, wird die Polarität der Elektroden gewechselt und ein elektrisches Gegenfeld aufgebaut. Hierdurch bedingt, wird die Wirkstoffabgabe gestoppt. Es ist nicht in jedem Fall erforderlich, daß das aufgebaute elektrische Gegenfeld bis zum nächsten Applikationszyklus, d.h. der nächsten Wirkstoffabgabe, bestehen bleibt. Nach einiger Zeit kann der Stromkreis zwischen der Spannungsquelle und den Elektroden vollständig unterbrochen werden, ohne daß weiterer Wirkstoff - mit Ausnahme der durch die passive Diffusion verursachte Anteil - durch die Haut penetriert. In der Vielzahl der Fälle ist es jedoch sinnvoll, das Gegenfeld bis zum nächsten Applikationszyklus aufrecht zu erhalten. Dadurch wird gewährleistet, daß kein weiterer Wirkstoff - auch nicht durch passive Diffusion - aufgenommen wird. Dies ist vor allem bei solchen Wirkstoffen von Bedeutung, die von der Haut gespeichert werden, d. h. die Haut selbst als Wirkstoffdepot wirkt.

In einer bevorzugten Ausführungsform besteht das erfindungsgemäße System aus einem Armband, das um das Handgelenk getragen wird. Auf der oberen Seite sind in einem miniaturisierten Gehäuse (vergleichbar mit den Abmessungen einer Uhr) eine oder mehrere Spannungsquellen, beispielsweise in Form von miniaturisierten Batterien, wie sie im Handel erhältlich sind, die integrierten Schaltungen für die Zeitkontrolle, für die Kontrolle der elektrischen Funktionen (Spannung, Frequenz, Höhe und Konstanz des elektrischen Feldes, sowie integrierte Schaltungen zum Umpolen des elektrischen Feldes vorhanden. Auf der Unterseite des Gehäuses ist die wirkstoffhaltige Elektrode angebracht, die einerseits elektrisch mit der Steuerungseinheit und der Stromquelle im Gehäuse verbunden ist und andererseits in Kontakt mit der Haut steht. Das Reservoir der Elektrode, das den Wirkstoff enthält, ist austauschbar. Entsprechend geeignete Konstruktionen, die einen Wirkstoff speichern können und eine elektrische Leitfähigkeit aufweisen, sind aus dem Stand der Technik bekannt.

Die zweite passive Elektrode ist so an dem Armband befestigt, daß sie am Handgelenk genau gegenüber der ersten Elektrode angeordnet werden kann. Diese zweite Elektrode ist ebenfalls elektrisch leitend mit der Steuerungseinheit und der Energieversorgung verbunden. Die passive Elektrode besteht entweder aus Metall oder anderen elektrisch leitenden, aber chemisch neutralen Stoffen.

Man unterscheidet bei der iontophoretischen und physiologischen Applikation von Wirkstoffen zwischen folgenden Arten der elektrischen Größen
a) konstante Gleichströme bzw. Gleichfelder
b) gepulste Gleichströme bzw. gepulste Gleichfelder unterschiedlichster Frequenz und Amplitudenform.

Die Verwendung gepulster Gleichströme ist beispielsweise in der Europäischen Patentschrift 138 347 beschrieben. Geeignete iontophoretische Systeme, die entsprechend der Lehre der vorliegenden Erfindung modifiziert werden können, sind beispielsweise in der Europäischen Patentanmeldung 225 872 bzw. 461 680 beschrieben. Insbesondere wird auf die Europäische Patentanmeldung 60 452 inhaltlich Bezug genommen.

Erfindungsgemäß wird bei der Wirkstoffapplikation ein gepulstes Gleichfeld definierter Frequenz bevorzugt, während zum Stoppen des Wirkstofffluxes ein elektrisches Gegenfeld mit bevorzugt konstanter Amplitude notwendig ist.

Im allgemeinen sollte die Amplitude des Gegenfeldes mindestens die gleiche Größenordnung aufweisen, wie die des Wirkfeldes in der Wirkstoff-Applikationsphase. Ein schnellerer Abbruch der Diffusion des Wirkstoffes wird dann erreicht, wenn die Stärke des Gegenfeldes insbesondere zu Beginn der Polarisationsumkehr größer ist, als das ursprünglich angelegte Feld zur Wirkstoffapplikation. Bevorzugt ist, wenn die Amplitude des Gleichfeldes umgekehrter Polarität (Gegenfeld) die 1,5 bis 2-fache Höhe der Amplitude des Feldes aufweist, bei der der Wirkstoff appliziert wird.

Bei Wirkstoffen, die ein sehr hohes passives Diffusionsvermögen aufweisen, kann es erforderlich sein, daß das Gegenfeld über einen längeren Zeitraum aufrechterhalten werden muß, möglicherweise während der ganzen Zeit, zu der kein Wirkstoff appliziert werden soll, um eine weitere unerwünschte Diffusion von Wirkstoff in das Blutgefäßsystem zu vermeiden. In manchen Fällen kann es jedoch ausreichend sein, wenn nach einem anfänglichen Gegenfeld mindestens gleicher Größenordnung ein wesentlich schwächeres Gegenfeld als das zuvor angelegte Feld dann über einen längeren Zeitraum aufrechterhalten wird.

Ein wesentlichor Aspekt bei miniaturisierten Iontophoresegeräten ist die Energieversorgung. Diesbezüglich ist es sinnvoll, das Gegenfeld nur so lange wie nötig zur Unterbrechung des Wirkstoffflusses eingeschaltet zu lassen und gegebenenfalls erneut für eine kurze Zeit einzuschalten. Die Diffusion des Wirkstoffes aus der Elektrode kann elektronisch gesteuert werden.

Wirkstoffhaltige auswechselbare Elektroden, wie sie in tragbaren Iontophoresegeräten Verwendung finden, sind aus dem Stand der Technik bekannt. In einer weiteren Ausführungsform kann die wirkstoffhaltige Elektrode eine separate Batterie aufweisen.

So beschreibt die deutsche Offenlegungsschrift 1965 146 leitfähige wirkstoffhaltige Gelkissen für die Iontophorese. Weiter entwickelte Elektroden sind beispielsweise aus der Europäischen Patentanmeldung 29 297 oder 252 732 bekannt.

In einer bevorzugten Ausführungsform besteht die wirkstoffhaltige Elektrode aus einer Elektrodenplatte und einem darauf befestigten Wirkstoffreservoir. Dieses Wirkstoffreservoir kann beispielsweise aus einem wirkstoffhaltigen Gel bestehen; auch ist es möglich, den Wirkstoff in einem Polymer zu inkorporieren. Geeignete Polymere hierfür sind beispielsweise nicht ionische polare synthetische Polymere, wie z.B. Poly(acrylamid), Poly(2-hydroxyethylacrylat, Poly(n-vinyl-2-pyrrolidon), Poly(n-methylolacrylamid), Poly(diacetonacrylamid), Poly(2-hydroxyethylmethacrylat), Poly(2-hydroxypropylmethacrylat), Poly(vinylalkohol), Poly(ethylenoxid), Poly(propylenoxide) und Poly(allylalkohol). Ebenso geeignet sind hydroxyfunktionalisierte Kondensationspolymere wie beispielsweise Polyester, Polycarbonate und Polyurethane als Vertreter der Gruppe der nichtionischen polaren Polymere.

Weiterhin geeignet sind Polyimide, Polysiloxane, Polyethylenglycolpolymer, Polyvinylpyrrolidone.

Weiterhin kann das Wirkstoffreservoir auch aus einem Gel bestehen, das beispielsweise Karayagummi oder ein Polysaccharid enthält.

Iontophoretische Systeme in Form eines Hautpflasters sind aus zahlreichen Europäischen Patentanmeldungen bekannt, so z.B. aus 147 524, 178 601, 225 556, 225 871, 230 749, 240 593, 269 246 und 293 893. Die dort beschriebenen Pflaster können in einfacherweise so modifiziert werden, daß sie für das erfindungsgemäß beanspruchte Verfahren genutzt werden können.

Als Wirkstoffe sind polare Verbindungen und insbesondere solche in ionisierbarer Form vorliegende geeignet. Geeignete Wirkstoffe für die iontophoretische Applikation sind beispielsweise Antiasthmatika, Broncholytika, Analgetika, Tranquilizer, Mittel zur Behandlung von Coronarerkrankungen, Antiparkinsonmittel, Wirkstoff zur Behandlung der Schizophrenie. Von besonderem Interesse ist auch die Applikation von Peptiden, wie z.B. LHRH, Enkephalin, Endorphin Interferon, Insulin, Carcitonin, TRN, NT-36 (chemical name: N-[[(s)-4-oxo-2-azetidinyl]carbonyl]-L-histidyl-L-prolinamide), Oxytocin, Liprecin, Vasopressin, Glucagon, Pituitary hormones (e.g., HGH, HMG, HCG, Desmopressin acetate.,), Follicle luteoids.

Die Konzentration an Wirkstoff in dem Wirkstoffreservoir beträgt zwischen 1 und 20 Gew.- %

### Beispiele:

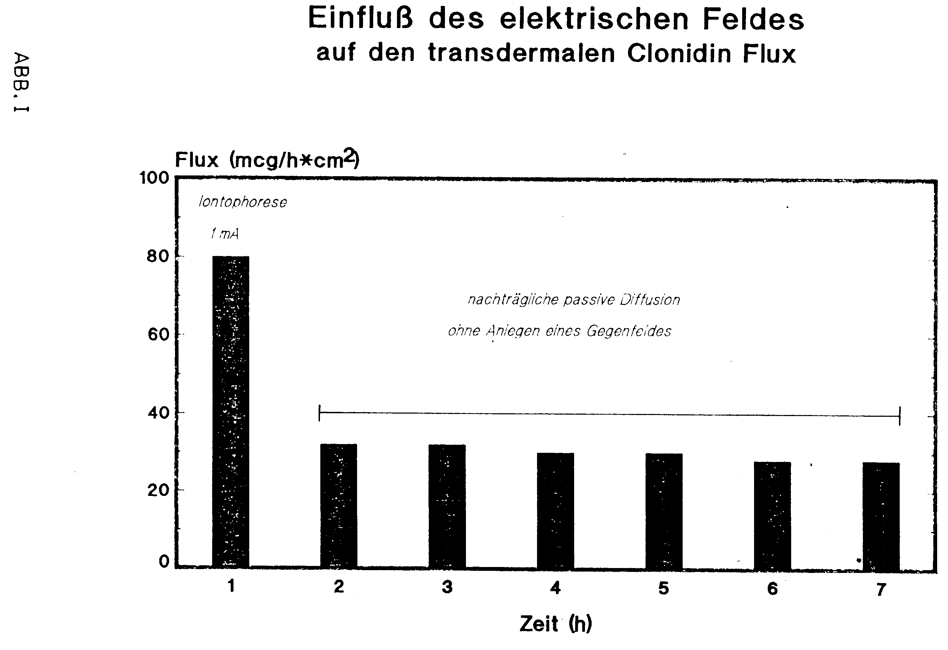

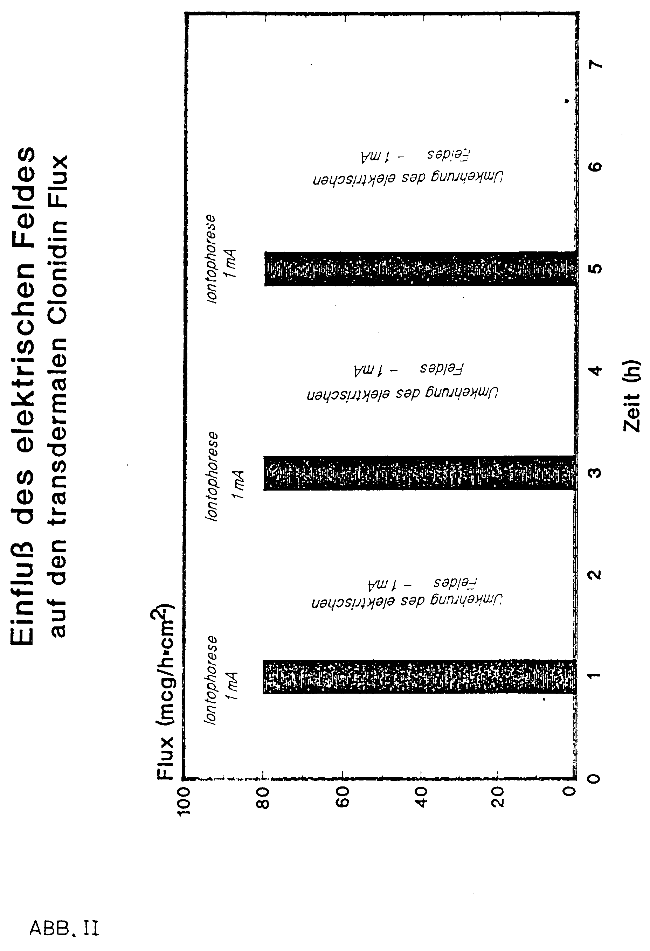

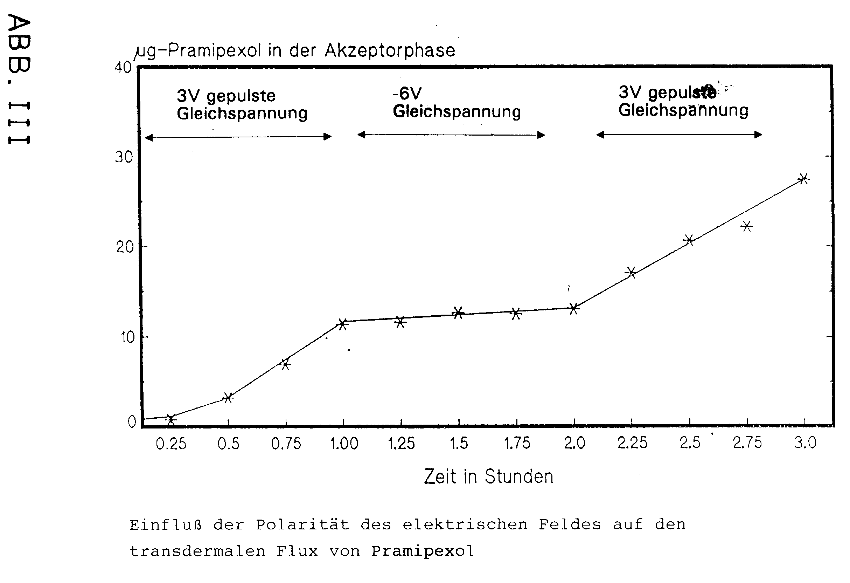

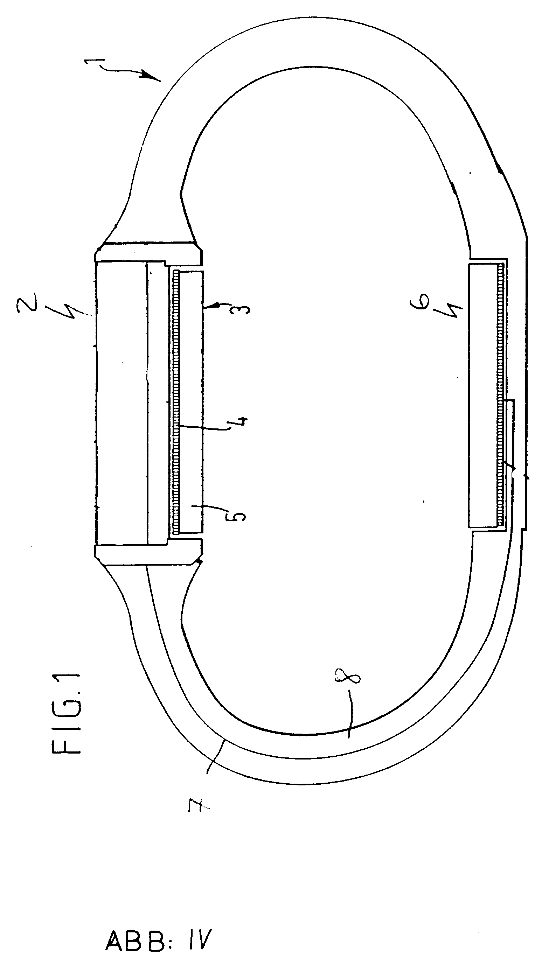

Die Abbildung 1 beschreibt die Fluxrate von Clonidin bei der iontophoretischen Applikation gemäß dem Stand der Technik.

Zum Zeitpunkt t = 1 Stunde ist ein nichtgepulstes Gleichfeld mit einer Stromstärke von 1 mA angelegt. Die Fluxrate beträgt 80 mcg/h cm². Zum Zeitpunkt t = 2 Stunden ist das elektrische Feld abgeschaltet, die Fluxrate beträgt ca. 38 mcg/h cm².

Infolge der passiven Diffusion der Wirkstoffe sinkt die Fluxrate auch zum Zeitpunkt t = 5 Stunden nur unwesentlich auf 30 mcg/h cm².

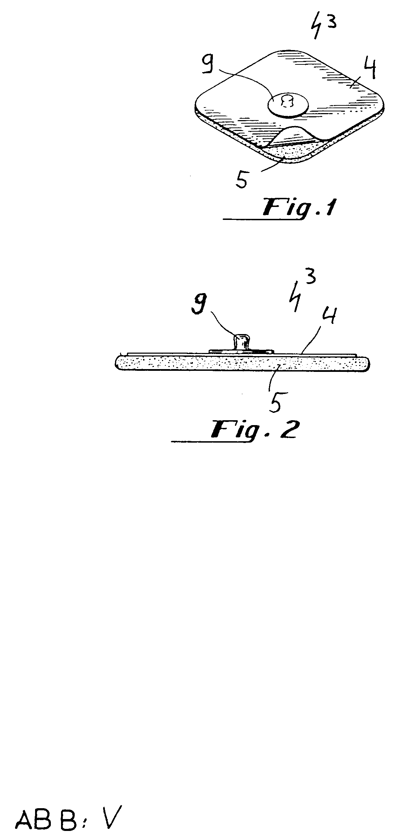

Abbildung 2 beschreibt die Fluxrate von Clonidin bei der iontophoretischen Applikation gemäß der Erfindung.

Im Gegensatz zur Abbildung 1 ist die Fluxrate zwischen den einzelnen Applikationsphasen - also zu t = 2, 4, 6 und 7 Stunden - nicht mehr meßbar, während sie zu den Zeitpunkten t = 1, 3 und 5 (Applikation bei 1 m A) konstant gleich hoch ist.

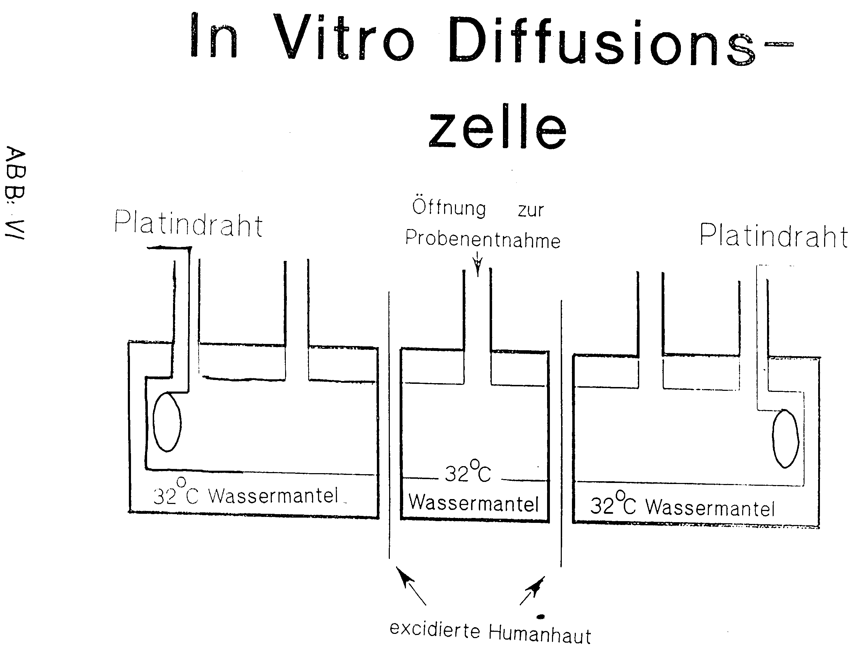

In Abbildung 3 ist der transdermale Fluß von Pramipexol in Abhängigkeit der angelegten Spannung dargestellt. Es wurde mit gepulster Gleichspannung gearbeitet, die ein "an/aus" Verhältnis von 1 : 1 hatte. In der ersten Stunde wurde eine positive Spannung von 3 Volt an die Elektrode der Donatorzelle angelegt. Der Wirkstoff wandert von der Donatorzelle durch die Haut in die Akzeptorzelle. In der zweiten Stunde wurde eine konstante Gegenspannung von 6 V zur Erzeugung des Gegenfeldes an die Elektroden angelegt.

In Abbildung VI ist das in-vitro Modell dargestellt, mit dem obige Versuche durchgeführt wurden. In dem Modell sind zwei menschliche Häute gegenüberliegend eingespannt. Der Wirkstoff Pramipexol wurde in ein Donatorkompartiment in Lösung eingefüllt. Der Gehalt an Pramipexol aus der mittleren Probenentnahme wurde durch HPLC bestimmt.

Die angelegte Spannung zur Erzeugung des. Gegenfeldes war in diesem Fall doppelt so groß wie die des zuvor angelegten gepulsten Gleichfeldes. Man erkennt, daß kein weiterer Wirkstoff migriert.

Eine Ausführungsform der vorliegenden Erfindung ist in Abb. IV dargestellt. Die iontophoretische Vorrichtung 1 besteht aus einem Gehäuse 2, an dessen Unterseite sich die wirkstoffhaltige Elektrode 3 befindet. Die passive - wirkstofffreie Elektrode 6 ist über einen elektrisch leitenden Kontakt 7 am Armband 8 mit dem Gehäuse verbunden. Die Batterie, die integrierte Schaltungen für Zeitkontrolle, Kontrolle der elektrischen Funktionen (Spannung, Frequenz, Größe und Konstanz des elektrischen Feldes, sowie integrierte Schaltungen zum Umpolen von einem gepulsten Gleichfeld zu einem konstanten Gleichfeld umgekehrter Polarität) sind im Gehäuse 2 integriert, aber in der Abbildung nicht dargestellt.

Die wirkstoffhaltige Elektrode 3 besteht aus dem eigentlichen Wirkstoffreservoir 5 und einer darüber angebrachten leitenden Schicht 4, die über einen Kontakt mit dem Gehäuse verbunden ist. Dieser Kontakt stellt gleichzeitig die elektrisch leitende Verbindung zur Stromversorgung und den integrierten Schaltungen im Gehäuse her. Das Wirkstoffreservoir und die Schicht 4 können mit einem elektrisch leitenden Kleber verbunden sein. Die Elektrode 6 besteht aus einem elektrisch leitenden Material.

In Abbildung V ist in Figur 1 die wirkstoffhaltige Elektrode 3 von oben mit Sicht auf den Kontakt 9 dargestellt. In Figur 2 ist ein Querschnitt der Elektrode dargestellt. Das Wirkstoffreservoir besteht aus einem Gelkissen oder einem Polymer, in dem der Wirkstoff inkorporiert ist. Vor Gebrauch ist das Wirkstoffreservoir durch eine abziehbare Schutzfolie abgedeckt.

## Patentansprüche

1. Verfahren zur kontrollierten Applikation von Wirkstoffen mittels Iontophorese, bei dem ein geeigneter Wirkstoff für eine vorherbestimmte Zeit unter Anwendung eines gegebenenfalls gepulsten elektrischen Gleichfeldes perkutan appliziert wird, dadurch gekennzeichnet, daß im Anschluß daran ein nicht gepulstes entgegengesetzt geladenes (entgegengerichtetes) elektrisches Gleichfeld (Gegenfeld) angelegt wird, wodurch die weitere Diffusion von Wirkstoff gestoppt wird.

2. Verfahren zur gezielten, schnelleren Unterbrechung des Wirkstoffflusses bei der Iontophorese, dadurch gekennzeichnet, daß ein elektrisches Gleichspannungsfeld angelegt wird, dessen Polarität der Migrationsrichtung des Wirkstoffes entgegengerichtet ist.

3. Verfahren nach Anspruch 1 oder 2, dadurch gekennzeichnet, daß die Höhe der Amplitude des nicht gepulsten entgegengerichteten Gleichfeldes (Gegenfeld) das 1,5 bis 2-fache der Höhe der Amplitude des gepulsten Gleichfeldes beträgt.

4. Vorrichtung zur kontrollierten Applikation von Wirkstoffen mittels Iontophorese, enthaltend
a) eine wirkstoffhaltige Elektrode,
b) eine wirkstofffreie Gegenelektrode,
c) eine Energiequelle zur Stromversorgung, die mit den beiden Elektroden verbunden ist, so daß bei angelegter Spannung zwischen den beiden Elektroden ein elektrisches Feld aufgebaut wird, wobei die Elektroden in Kontakt mit der Haut des Patienten sind,
dadurch gekennzeichnet, daß sie
d) eine Vorrichtung zur Umkehrung der Polarität der Elektroden aufweist, so daß nach der Polaritätsumkehr der Wirkstofffluss gestoppt wird.

5. Vorrichtung nach Anspruch 3, dadurch gekennzeichnet, daß sie
a) eine integrierte Schaltung zur Erzeugung eines gepulsten Gleichfeldes und
b) eine integrierte Schaltung zur Erzeugung eines Gleichfeldes umgekehrter Polarität (Gegenfeld)
enthält.

6. Vorrichtung nach Anspruch 5, dadurch gekennzeichnet, daß das Gegenfeld eine konstante Amplitudenhöhe aufweist.
